(19) **Europäisches Patentamt / European Patent Office / Office européen des brevets**

(11) **EP 4 289 277 A1**

(12) **EUROPEAN PATENT APPLICATION**
published in accordance with Art. 153(4) EPC

(43) Date of publication:
**13.12.2023 Bulletin 2023/50**

(21) Application number: **22749825.0**

(22) Date of filing: **04.02.2022**

(51) International Patent Classification (IPC):
**A23C 19/032** (2006.01)      **A23C 19/068** (2006.01)
**C12N 9/20** (2006.01)      **C12N 9/62** (2006.01)

(52) Cooperative Patent Classification (CPC):
**A23C 19/032; A23C 19/068; C12N 9/20; C12N 9/62**

(86) International application number:
**PCT/JP2022/004515**

(87) International publication number:
**WO 2022/168954 (11.08.2022 Gazette 2022/32)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **04.02.2021   JP 2021016920**

(71) Applicants:
- **Amano Enzyme U.S.A. Co., Ltd.**
  **Elgin, Illinois 60124 (US)**
- **Amano Enzyme Inc.**
  **Nagoya-shi**
  **Aichi 460-8630 (JP)**

(72) Inventors:
- **OKUDA, Keita**
  **Kakamigahara-shi, Gifu 509-0109 (JP)**
- **NAGAYA, Miho**
  **Kakamigahara-shi, Gifu 509-0109 (JP)**

(74) Representative: **Müller-Boré & Partner
Patentanwälte PartG mbB
Friedenheimer Brücke 21
80639 München (DE)**

Remarks:
The complete document including Reference Table(s) and the Sequence Listing(s) can be downloaded from the EPO website

(54) **NATURAL CHEESE PRODUCTION METHOD**

(57)    The purpose of the present invention is to provide a technology for promoting short-chain fatty acid separation in a natural cheese production technology employing lipase of microbial origin. It is possible to promote short-chain fatty acid separation by means of a natural cheese production method including a step for causing lipase of microbial origin and protease of filamentous fungal origin to act on milk.

EP 4 289 277 A1

**Description**

TECHNICAL FIELD

The present invention relates to a production method of a natural cheese.

BACKGROUND ART

Lipases are enzymes that catalyze the hydrolysis of ester bonds in lipid substrates, and bring about the release of fatty acids. In dairy industry applications, the lipases are utilized in the flavor production and/or flavor enhancement of dairy products and play an important role, especially in the production of cheese.

As a lipase used for producing cheese, lipases derived from ruminants such as kids, calves, and lambs have been traditionally used. The ruminant-derived lipase is excellent in the property of releasing short-chain fatty acids (particularly, butyric acid having 4 carbon atoms) from milk fat, and this excellent property has been used for the flavor production and/or flavor enhancement of cheese for a long time.

On the other hand, there is a strong industrial need for an alternative to animal-derived lipases because kosher or halal qualities are required for enzyme preparations utilized for food product processing. To meet the need, proposals have been made to use microbe-derived enzymes (for example, Patent Document 1), recombinant enzymes (for example, Patent Document 2), and the like. Attempts have also been made to modify lipases by genetic engineering, for application to particular purposes (for example, Patent Documents 3 to 5), and lipases in which a mutation imparting short-chain fatty acid selectivity to a specific microbe-derived lipase having a low ability to release short-chain fatty acids by increasing the ability to release short-chain fatty acids is introduced, have also been proposed (Patent Documents 6 and 7).

PRIOR ART DOCUMENTS

PATENT DOCUMENTS

Patent Document 1: Japanese Patent Laid-open Publication No. S61-135541
Patent Document 2: US Patent Application Publication No. 2004/0001819
Patent Document 3: Japanese Translation of PCT International Publication No. 2011-512809
Patent Document 4: Japanese Translation of PCT International Publication No. 2003-524386
Patent Document 5: Japanese Translation of PCT International Publication No. 2004-517639
Patent Document 6: PCT International Publication No. 2015/087833
Patent Document 7: PCT International Publication No. 2017/211930

SUMMARY OF THE INVENTION

PROBLEMS TO BE SOLVED BY THE INVENTION

The present inventors have considered that the ruminant-derived lipase traditionally used in the production of a natural cheese can be substituted for a microbe-derived lipase having short-chain fatty acid selectivity. However, even when the microbe-derived lipase is used in the production of a natural cheese, contrary to the expectation, a new problem is encountered in that the ability to release short-chain fatty acids is not sufficiently exerted.

Therefore, an object of the present invention is to provide a technology for promoting short-chain fatty acid release in a natural cheese production technology using a microbe-derived lipase.

MEANS FOR SOLVING THE PROBLEM

The present inventors have considered that the reason why the ability to release short-chain fatty acids is not exhibited even when a microbe-derived lipase is used in the production of a natural cheese is an environment in which the lipase cannot react with fat globules. It is usual to use an emulsifier for the destruction of fat globules, but since there is a restriction that the emulsifier is not used in the production of a natural cheese, the emulsifier cannot be used. In this regard, the present inventors have conducted intensive studies, and as a result, have found that by using a filamentous fungus-derived protease in combination with a lipase, the lipase easily acts and the release of short-chain fatty acids is promoted.

That is, the present invention provides inventions of the following aspects.

Item 1. A production method of a natural cheese including a step of causing a microbe-derived lipase and a filamentous fungus-derived protease to act on milk.

Item 2. The production method described in item 1, in which the microbe-derived lipase comprises at least any one of polypeptides shown in (1) to (3) below:

(1) a polypeptide comprising an amino acid sequence in which amino acid sequences at the 1st to 15th positions are removed from an amino acid sequence shown in any one of SEQ ID NOs: 1 to 15 or each of amino acid sequence shown in SEQ ID NOs: 1 to 15;

(2) a polypeptide comprising an amino acid sequence in which amino acid sequences at the 1st to 15th positions are removed from an amino acid sequence shown in any one of SEQ ID NOs: 1 to 15 or each of amino acid sequence shown in SEQ ID NOs: 1 to 15 obtained by substituting, adding, inserting, or deleting one or several amino acid residues and having short-chain fatty acid selectivity; and

(3) a polypeptide having 70% or more sequence identity to an amino acid sequence in which amino acid sequences at the 1st to 15th positions are removed from an amino acid sequence shown in any one of SEQ ID NOs: 1 to 15 or each of amino acid sequence shown in SEQ ID NOs: 1 to 15, and having short-chain fatty acid selectivity.

Item 3. The production method described in item 1 or 2, in which a used amount of the microbe-derived lipase is 1 LU or more per 1 g of milk fat in the milk.

Item 4. The production method described in any one of items 1 to 3, in which a used amount of the filamentous fungus-derived protease per 1 LU of the microbe-derived lipase is 0.02 U or more.

Item 5. The production method described in any one of items 1 to 4, in which a used amount of the filamentous fungus-derived protease is 0.3 U or more per 1 g of milk protein in the milk.

Item 6. The production method described in item 5, in which the used amount of the filamentous fungus-derived protease is 20 U or less per 1 g of milk protein in the milk.

Item 7. The production method described in any one of items 1 to 6, in which the filamentous fungus-derived protease is a protease derived from the genus Aspergillus.

Item 8. A natural cheese comprising a microbe-derived lipase and a filamentous fungus-derived protease.

Item 9. A short-chain fatty acid release promoter in natural cheese production using a microbe-derived lipase, comprising a filamentous fungus-derived protease.

Item 10. A production method of a processed cheese product, including: a step of producing a natural cheese by the production method described in any one of items 1 to 7; and a step of processing the natural cheese.

Item 11. A processed cheese product produced by the production method described in item 10.

ADVANTAGES OF THE INVENTION

According to the present invention, there is provided a technology for promoting short-chain fatty acid release in a natural cheese production technology using a microbe-derived lipase.

BRIEF DESCRIPTION OF THE DRAWINGS

Fig. 1 shows a free fatty acid composition in a natural cheese obtained in Test Example 1. Six bas graphs showing the released amount of each fatty acid show the released amounts according to Comparative Example 1, Reference Example 1, Comparative Example 2, Example 1, Example 2, and Comparative Example 3.
Fig. 2 shows a free fatty acid composition in a natural cheese obtained in Test Example 2.

EMBODIMENTS OF THE INVENTION

Hereinafter, the present invention will be described in detail. 20 amino acid residues in amino acid sequences excluding the sequence lists may be abbreviated as one-letter codes. That is, glycine (Gly) is G, alanine (Ala) is A, valine (Val) is V, leucine (Leu) is L, isoleucine (Ile) is I, phenylalanine (Phe) is F, tyrosine (Tyr) is Y, tryptophan (Trp) is W, serine (Ser) is S, treonine (Thr) is T, cysteine (Cys) is C, methionine (Met) is M, aspartic acid (Asp) is D, glutamic acid (Glu) is E, asparagine (Asn) is N, glutamine (Gln) is Q, lysine (Lys) is K, arginine (Arg) is R, histidine (His) is H, and proline (Pro) is P.

In the present specification, the amino acid sequence to be displayed is N-terminal at the left end and C-terminal at the right end.

In the present specification, the term "non-polar amino acid" includes alanine, valine, leucine, isoleucine, proline, methionine, phenylalanine, and tryptophan. The term "non-charged amino acid" includes glycine, serine, threonine, cysteine, tyrosine, asparagine, and glutamine. The term "acidic amino acid" includes aspartic acid and glutamic acid. The term "basic amino acid" includes lysine, arginine, and histidine.

In the present specification, the term "substitution" includes not only a case where a substitution of an amino acid residue is artificially introduced, but also a case where a substitution of an amino acid residue is naturally introduced, that is, a case where amino acid residues are originally different. In the present specification, the substitution of an amino acid residue may be an artificial substitution or a natural substitution, but an artificial substitution is preferred.

1. Production Method of Natural Cheese

A production method of a natural cheese of the present invention includes a step of causing a microbe-derived lipase and a filamentous fungus-derived protease to act on milk. Hereinafter, the production method of a natural cheese of the present invention will be specifically described.

1-1. Natural Cheese

In the present invention, the natural cheese belongs to a type of cheese obtained by aging a card of milk, and does not contain an emulsifier. The natural cheese may be any of soft cheese, semi-hard cheese, and hard/ultra-hard cheese (hard cheese.

1-2. Milk

As the type of the milk, milk used for normal cheese production is used without particular limitation. Specific examples of the milk include cow milk, goat milk, buffalo milk, and sheep milk. These kinds of milk may be used singly or in combination of a plurality of kinds thereof. Among these kinds of milk, cow milk is preferable.

1-3. Microbe-derived lipase

The microbe-derived lipase used in the present invention includes both a natural lipase found in microorganisms and a lipase artificially modified based on the sequence of the natural lipase as a basic skeleton as long as the microbe-derived lipase has short-chain fatty acid selectivity.

In the present invention, the "short-chain fatty acid selectivity" refers to a property that, among fatty acids to be released from lipids, the released amount of butyric acid having 4 carbon atoms is higher (compared on a molar basis) as compared to each fatty acid having 5 or more carbon atoms. Specifically, the "short-chain fatty acid selectivity" can be confirmed by the fact that the released amount of the C4 fatty acid (butyric acid) is larger than the released amount of each of the C6, C8, C10, C12, C14, C16, C18, and C18:1 fatty acids (compared on a molar basis) in the case of measuring the released amounts of C4, C6, C8, C10, C12, C14, C16, C18, and C18: 1 fatty acids after using 2 g of milk fat (in a state where fat globules are destructed and milk fat is exposed) as a substrate and subjecting the polypeptide (lipase) to a condition of acting in an amount of 3 LU per 1 g of milk fat at 40°C for 30 minutes.

In the production method of the present invention, the microbe-derived lipase itself has a property of enhancing the ability to release short-chain fatty acids, but even when the microbe-derived lipase cannot exhibit the property only by replacing the ruminant-derived lipase in the production of a natural cheese, the microbe-derived lipase can exhibit the property by being used in combination with a filamentous fungus-derived protease.

Specific examples of the microbe-derived lipase used in the present invention include lipases comprising at least any one of polypeptides shown in (1) to (3) below:

(1) a polypeptide comprising an amino acid sequence in which amino acid sequences at the 1st to 15th positions are removed from an amino acid sequence shown in any one of SEQ ID NOs: 1 to 15 or each of amino acid sequence shown in SEQ ID NOs: 1 to 15;

(2) a polypeptide comprising an amino acid sequence in which amino acid sequences at the 1st to 15th positions are removed from an amino acid sequence shown in any one of SEQ ID NOs: 1 to 15 or each of amino acid sequence shown in SEQ ID NOs: 1 to 15 obtained by substituting, adding, inserting, or deleting one or several amino acid residues and having short-chain fatty acid selectivity; and

(3) a polypeptide having 70% or more sequence identity to an amino acid sequence in which amino acid sequences at the 1st to 15th positions are removed from an amino acid sequence shown in any one of SEQ ID NOs: 1 to 15 or each of amino acid sequence shown in SEQ ID NOs: 1 to 15, and having short-chain fatty acid selectivity.

The polypeptides shown in (1) to (3) have lipase activity and short-chain fatty acid selectivity.

Among the polypeptides of (1), the polypeptide comprising an amino acid sequence shown in SEQ ID NO: 1 is a known lipase which has a wild-type lipase LIP1 (including a signal peptide) derived from Candida cylindracea as a basic skeleton, and is mutated so as to have short-chain fatty acid selectivity by substituting A for P which is an amino acid residue at the 261st position of the amino acid sequence comprising 549 amino acid residues of LIP1.

Among the polypeptides of (1), the polypeptide comprising an amino acid sequence shown in SEQ ID NO: 2 is a known lipase which has the lipase LIP1 (including a signal peptide) as a basic skeleton, and is mutated so as to have short-chain fatty acid selectivity by substituting F for P which is an amino acid residue at the 261st position of the amino acid sequence comprising 549 amino acid residues of LIP1.

Among the polypeptides of (1), the polypeptide comprising an amino acid sequence shown in SEQ ID NO: 3 is a known lipase which has the lipase LIP1 (including a signal peptide) as a basic skeleton, and is mutated so as to have short-chain fatty acid selectivity by substituting L for P which is an amino acid residue at the 261st position of the amino acid sequence comprising 549 amino acid residues of LIP1.

Among the polypeptides of (1), the polypeptide comprising an amino acid sequence shown in SEQ ID NO: 4 is a known lipase which has the lipase LIP1 (including a signal peptide) as a basic skeleton, and is mutated so as to have short-chain fatty acid selectivity by substituting S for P which is an amino acid residue at the 261st position of the amino acid sequence comprising 549 amino acid residues of LIP1.

Among the polypeptides of (1), the polypeptide comprising an amino acid sequence shown in SEQ ID NO: 5 is a known lipase which has the lipase LIP1 (including a signal peptide) as a basic skeleton, and is mutated so as to have short-chain fatty acid selectivity by substituting W for L which is an amino acid residue at the 322nd position of the amino acid sequence comprising 549 amino acid residues of LIP1.

Among the polypeptides of (1), the polypeptide comprising an amino acid sequence shown in SEQ ID NO: 6 is a known lipase which has the lipase LIP1 (including a signal peptide) as a basic skeleton, and is mutated so as to have short-chain fatty acid selectivity by substituting L for F which is an amino acid residue at the 360th position of the amino acid sequence comprising 549 amino acid residues of LIP1.

Among the polypeptides of (1), the polypeptide comprising an amino acid sequence shown in SEQ ID NO: 7 is a known lipase which has the lipase LIP1 (including a signal peptide) as a basic skeleton, and is mutated so as to have short-chain fatty acid selectivity by substituting I for S which is an amino acid residue at the 380th position of the amino acid sequence comprising 549 amino acid residues of LIP1.

Among the polypeptides of (1), the polypeptide comprising an amino acid sequence shown in SEQ ID NO: 8 is a known lipase which has the lipase LIP1 (including a signal peptide) as a basic skeleton, and is mutated so as to have short-chain fatty acid selectivity by substituting L for S which is an amino acid residue at the 380th position of the amino acid sequence comprising 549 amino acid residues of LIP1.

Among the polypeptides of (1), the polypeptide comprising an amino acid sequence shown in SEQ ID NO: 9 is a known lipase which has the lipase LIP1 (including a signal peptide) as a basic skeleton, and is mutated so as to have short-chain fatty acid selectivity by substituting W for L which is an amino acid residue at the 425th position of the amino acid sequence comprising 549 amino acid residues of LIP1.

Among the polypeptides of (1), the polypeptide comprising an amino acid sequence shown in SEQ ID NO: 10 is a known lipase which has the lipase LIP1 (including a signal peptide) as a basic skeleton, and is mutated so as to have short-chain fatty acid selectivity by substituting F for L which is an amino acid residue at the 428th position of the amino acid sequence comprising 549 amino acid residues of LIP1.

Among the polypeptides of (1), the polypeptide comprising an amino acid sequence shown in SEQ ID NO: 11 is a known lipase which has the lipase LIP1 (including a signal peptide) as a basic skeleton, and is mutated so as to have short-chain fatty acid selectivity by substituting N for L which is an amino acid residue at the 428th position of the amino acid sequence comprising 549 amino acid residues of LIP1.

Among the polypeptides of (1), the polypeptide comprising an amino acid sequence shown in SEQ ID NO: 12 is a known lipase which has the lipase LIP1 (including a signal peptide) as a basic skeleton, and is mutated so as to have short-chain fatty acid selectivity by substituting F for G which is an amino acid residue at the 429th position of the amino acid sequence comprising 549 amino acid residues of LIP1.

Among the polypeptides of (1), the polypeptide comprising an amino acid sequence shown in SEQ ID NO: 13 is a known lipase which has the lipase LIP1 (including a signal peptide) as a basic skeleton, and is mutated so as to have short-chain fatty acid selectivity by substituting M for G which is an amino acid residue at the 429th position of the amino acid sequence comprising 549 amino acid residues of LIP1.

Among the polypeptides of (1), the polypeptide comprising an amino acid sequence shown in SEQ ID NO: 14 is a known lipase which has the lipase LIP1 (including a signal peptide) as a basic skeleton, and is mutated so as to have short-chain fatty acid selectivity by substituting I for G which is an amino acid residue at the 429th position of the amino acid sequence comprising 549 amino acid residues of LIP1.

Among the polypeptides of (1), the polypeptide comprising an amino acid sequence shown in SEQ ID NO: 15 is a known lipase which has the lipase LIP1 (including a signal peptide) as a basic skeleton, and is mutated so as to have short-chain fatty acid selectivity by substituting F for V which is an amino acid residue at the 549th position of the amino acid sequence comprising 549 amino acid residues of LIP1.

The amino acid sequences shown in SEQ ID NOs: 1 to 15 have extremely high sequence identity with each other. For example, with respect to the amino acid sequence shown in SEQ ID NO: 13, in the amino acid sequences of SEQ ID NOs: 12 and 14, only one amino acid residue is substituted, and in the amino acid sequences of SEQ ID NOs: 1 to 11 and 15, only two amino acid residues are substituted. In any of the amino acid sequences shown in SEQ ID NOs: 1 to 15, the amino acid sequences at the 1st to 15th positions form signal peptide sequences, and these signal peptide sequences completely coincide with each other.

Among the polypeptides of (1), the polypeptides comprising an amino acid sequence in which amino acid sequences (signal peptide sequences) at the 1st to 15th positions are removed from each of amino acid sequence shown in SEQ ID NOs: 1 to 15 are each a mature sequence of the amino acid sequences shown in SEQ ID NO: 1 to 15.

The polypeptides of (2) and (3) comprise an amino acid sequence having an amino acid sequence of the polypeptide of (1) as a basic skeleton. The polypeptides of (2) and (3) have lipase activity and short-chain fatty acid selectivity as with the polypeptide of (1), although there is a difference in amino acid sequence as compared with the polypeptide of (1).

The short-chain fatty acid selectivity of the polypeptides of (2) and (3) is not particularly limited as long as the above-described short-chain fatty acid selectivity is satisfied, but as an example of the degree of short-chain fatty acid selectivity, in the case of measuring the released amount (on a molar basis) of the C4 fatty acid obtained by subjecting the polypeptide to the above-described conditions and the total amount of released amounts (on a molar basis) of C6, C8, C10, C12, C14, C16, C18, and C18: 1 fatty acids, a ratio of the released amount of the C4 fatty acid to the total amount is 0.8 to 1.2 times the ratio for the corresponding polypeptide of (1). The "corresponding polypeptide of (1)" refers to a polypeptide comprising an amino acid sequence as a reference in the polypeptides of (2) and (3). As an example, when each of the polypeptides of (2) and (3) is "a polypeptide comprising an amino acid sequence shown in SEQ ID NO: 13 obtained by substituting, adding, inserting, or deleting one or several amino acid residues and having short-chain fatty acid selectivity" and "a polypeptide having 70% or more sequence identity to an amino acid sequence shown in SEQ ID NO: 13 and having short-chain fatty acid selectivity", the "polypeptide of (1) corresponding" to each polypeptide is "a polypeptide comprising an amino acid sequence shown in SEQ ID NO: 13."

The amino acid difference in the polypeptide of (2) may comprise only one of the differences (for example, substitution) including substitution, addition, insertion, and deletion or comprise two or more of the differences (for example, substitution and insertion). The number of amino acid differences in the polypeptide of (2) may be one or several, and is, for example, 1 to 50, preferably 1 to 20, 1 to 10, 1 to 8, 1 to 7, 1 to 6, 1 to 5, or 1 to 4, further preferably 1 to 3, and particularly preferably 1 or 2, or 1.

In the polypeptide of (3), the sequence identity to an amino acid sequence in which amino acid sequences at the 1st to 15th positions are removed from an amino acid sequence shown in any one of SEQ ID NOs: 1 to 15 or each of amino acid sequence shown in SEQ ID NOs: 1 to 15 may be 70% or more, and is preferably 80% or more, 85% or more or 90% or more, more preferably 95% or more, 96% or more, 97% or more, or 98% or more, and further preferably 99% or more, 99.4% or more, 99.6% or more, or 99.8% or more.

In the polypeptide of (3), the "sequence identity" refers to a value of amino acid sequence identity obtained by bl2seq program (Tatiana A. Tatsusova, Thomas L. Madden, FEMS Microbiol. Lett., Vol. 174, p 247-250, 1999) in BLAST PACKAGE [sgi32 bit edition, Version 2.0.12; available from National Center for Biotechnology Information (NCBI)]. Parameter settings may be as follows: Gap insertion Cost value: 11 and Gap extension Cost value: 1.

In the polypeptides of (2) and (3), since the amino acid residues at the 224th position (S), the 356th position (E), and the 464th position (H) in amino acid sequence shown in any one of SEQ ID NOs: 1 to 15 is considered to contribute to lipase activity, it is desirable not to introduce substitutions or deletions at these sites. Since amino acid residues at the 261st positions of SEQ ID NOs: 1, 2, 3, and 4, the 322nd position of SEQ ID NO: 5, the 360th position of SEQ ID NO: 6, the 380th positions of SEQ ID NOs: 7 and 8, the 425th position of SEQ ID NO: 9, the 428th position of SEQ ID NOs: 10 and 11, the 429th positions of SEQ ID NOs: 12, 13, and 14, and the 549th position of SEQ ID NO: 15 are considered to contribute to substrate specificity, it is desirable not to introduce substitutions or deletions at these sites. That is, in the polypeptide of (2), the site of the amino acid to be substituted, added, inserted, or deleted is desirably a site other than the site considered to contribute to the lipase activity and the site considered to contribute the substrate specificity, and in the polypeptide of (3), the sequence identity is desirably a sequence identity in a site excluding the site considered to contribute to the lipase activity and the site considered to contribute the substrate specificity.

When an amino acid substitution is introduced into the polypeptides of (2) and (3), a conservative substitution is mentioned as an aspect of the amino acid substitution. That is, examples of the amino acid substitution introduced in the polypeptides of (2) and (3) include the following substitutions: when an amino acid to be substituted is a non-polar amino acid, a substitution with other non-polar amino acids; when an amino acid to be substituted is a non-charged amino acid, a substitution with other non-charged amino acids; when an amino acid to be substituted is an acidic amino acid, a substitution with other acidic amino acids; and when an amino acid to be substituted is a basic amino acid, a substitution with other basic amino acids.

The polypeptides of (1) to (3) include not only polypeptides obtained by artificial substitution but also polypeptides originally having such an amino acid sequence.

The used amount of the microbe-derived lipase is not particularly limited as long as the desired effect of the present invention can be obtained, and is, for example, 1 LU or more per 1 g of milk fat in the milk used for a material. From the viewpoint of further enhancing the effect of promoting short-chain fatty acid release, the used amount of the microbe-derived lipase is preferably 1.4 LU or more, more preferably 2 LU or more, further preferably 2.5 LU or more, still more preferably 3 LU or more, even still more preferably 3.5 LU or more, and particularly preferably 4 LU or more per 1 g of milk fat in the milk. The upper limit of the used amount range of the microbe-derived lipase is not particularly limited, and is, for example, 20 LU or less, 10 LU or less, 8 LU or less, 5 LU or less, or 4.5 LU or less per 1 g of milk fat in the milk.

The lipase activity of the microbe-derived lipase is measured using tributyrin as a substrate by a lipase activity test method. That is, in the present invention, an amount of an enzyme which causes an increase in butyric acid of 1 $\mu$mol per minute when an enzymatic reaction is performed using tributyrin as a substrate by a conventional method is defined as lipase activity of 1 LU.

1-4. Filamentous Fungus-Derived Protease

The filamentous fungus-derived protease used in the present invention promotes the release of butyric acid, which is a short-chain fatty acid, as compared with the case of using a microbe-derived lipase alone without using a filamentous fungus-derived protease in combination. One of the mechanisms by which such an effect can be obtained is probably that the filamentous fungus-derived protease destructs fat globules, thereby facilitating the action of the microbe-derived lipase on milk fat.

The filamentous fungus-derived protease used in the present invention is distinguished from a filamentous fungus-derived protease, which is an example of a milk coagulating enzyme, in that the filamentous fungus-derived protease does not have a milk coagulating activity. That is, the milk coagulating enzyme is excluded from the filamentous fungus-derived protease used in the present invention.

The type of the filamentous fungus-derived protease is not limited to serine protease, metal protease, thiol protease, and aspartic protease (acid protease) in the classification based on the catalytic mechanism, but metal protease is preferable. The type of the filamentous fungus-derived protease is not limited to acid protease, neutral protease, and alkaline protease in the classification based on the optimum pH, but neutral protease is preferable.

The filamentous fungus-derived protease is not particularly limited as long as it can obtain the desired effect of the present invention and can be used for food products. Specific examples of the filamentous fungus-derived protease include proteases derived from the genera Aspergillus, Mucor, Neurospora, Penicillium, Rhizomucor, Rhizopus, and Sclerotinia. These filamentous fungus-derived proteases may be used singly or in combination of a plurality of kinds thereof. Among these filamentous fungus-derived proteases, from the viewpoint of further enhancing the effect of promoting short-chain fatty acid release, a protease derived from the genus Aspergillus is preferable.

Specific examples of the protease derived from the genus Aspergillus include proteases derived from Aspergillus oryzae, Aspergillus niger, Aspergillus melleus, Aspergillus japonicus, Aspergillus awamori, Aspergillus kawachii, Aspergillus sojae, Aspergillus tamarii, Aspergillus foetidus, Aspergillus fumigatus, Aspergillus nidulans, Aspergillus aculeatus, Aspergillus candidus, Aspergillus flavus, Aspergillus saitoi, Aspergillus inuii, Aspergillus glaucus, Aspergillus caesiellus, Aspergillus clavatus, Aspergillus deflectus, Aspergillus fischerianus, Aspergillus parasiticus, Aspergillus penicilloides, Aspergillus restrictus, Aspergillus sydowii, Aspergillus terreus, Aspergillus ustus, and Aspergillus versicolor. These proteases derived from the genus Aspergillus may be used singly or in combination of a plurality of kinds thereof. Among these proteases derived from the genus Aspergillus, from the viewpoint of further enhancing the effect of promoting short-chain fatty acid release, a protease derived from Aspergillus oryzae is preferable.

The filamentous fungus-derived protease can be prepared by a known method. As an example, when the protease derived from the genus Aspergillus is prepared, the protease can be easily prepared by a method of producing a microbe belonging to the genus Aspergillus and separating the protease using a known means, a method using a genetic recombination technique, or the like. As the filamentous fungus-derived protease. a commercially available product may be used. Examples of the commercially available filamentous fungus-derived protease include Protease M "Amano" (acid protease derived from Aspergillus oryzae), Protease A "Amano" (neutral protease derived from Aspergillus oryzae), and Protease A "Amano" 2SD (neutral protease derived from Aspergillus oryzae) manufactured by Amano Enzyme Inc.; and Sumizyme MP (alkaline protease derived from Aspergillus melleus) and Sumizyme FL-G (alkaline protease derived from Aspergillus oryzae) manufactured by Shinnihon Chemicals Co., Ltd.

The filamentous fungus-derived protease is more preferable as the ratio of peptidase activity (exopeptidase activity) to protease activity (total protease activity) is higher from the viewpoint of further enhancing the effect of promoting short-chain fatty acid release.

The used amount of the filamentous fungus-derived protease is not particularly limited as long as the desired effect of the present invention can be obtained, and is, for example, 0.3 U or more per 1 g of milk protein in the milk used for a material. From the viewpoint of further enhancing the effect of promoting short-chain fatty acid release, the used amount of the filamentous fungus-derived protease is preferably 0.6 U or more, more preferably 0.7 U or more, further preferably 0.8 U or more, and still more preferably 0.9 U or more, 1.5 U or more, 2 U or more, 2.5 U or more, or 3 U or more per 1 g of milk protein in the milk. The upper limit of the used amount range of the filamentous fungus-derived protease is not particularly limited, and is, for example, 20 U or less per 1 g of milk protein in milk, and from the viewpoint of improving the shape retention of a natural cheese to be obtained, the upper limit thereof is preferably 10 U or less, more preferably 5 U or less, further preferably 3 U or less, still more preferably 2 U or less, even still more preferably 1.5 U or less, and particularly preferably 1.2 U or less per 1 g of milk protein in the milk used for a material.

The ratio of the used amount of the filamentous fungus-derived protease to the used amount of the microbe-derived lipase is not particularly limited as long as the desired effect of the present invention is obtained, but from the viewpoint of further enhancing the effect of promoting short-chain fatty acid release, the used amount of the protease is, for example, 0.02 U or more, preferably 0.1 U or more, more preferably 0.15 U or more, further preferably 0.8 U or more, still more preferably 2 U or more, and even still more preferably 5 U or more, 50 U or more, or 150 U or more per 1 LU of the microbe-derived lipase. The upper limit of the ratio range of the used amount of the filamentous fungus-derived protease to the used amount of the microbe-derived lipase is not particularly limited, and is, for example, 300 U or less or 200 U or less.

The filamentous fungus-derived protease activity is measured using casein as a substrate by the Folin method. That is, in the present invention, an amount of an enzyme which causes an increase in colored materials by Folin's reagent corresponding to 1 μg of tyrosine per minute when an enzymatic reaction is performed using casein as a substrate by a conventional method is defined as filamentous fungus-derived protease activity of 1 U.

1-5. Milk coagulating enzyme

In the present invention, in addition to the above enzyme, a milk coagulating enzyme is used. As the milk coagulating enzyme, a known milk coagulating enzyme can be used without particular limitation, and examples thereof include rennet derived from ruminants of suckling period such as kids, calves, or lambs; rennet derived from microorganisms such as yeast, basidiomycetes, filamentous fungi, and bacteria; genetically modified rennet (genetically modified chymosin); and rennet derived from plants such as ficin of ficus, papain of papaya, and bromelain of pineapple.

As the used amount of the milk coagulating enzyme, a person skilled in the art can appropriately select a normal amount to be used in the case of coagulating milk in the natural cheese production process.

1-6. Procedures, Reaction Conditions, etc. and Other Steps

A specific procedure in the production method of a natural cheese of the present invention may be set so that lactic acid fermentation of milk, coagulation precipitation (carding) of casein molecules by a milk coagulating enzyme, and short-chain fatty acid release by the filamentous fungus-derived protease and the microbe-derived lipase are performed in a timely manner.

The timing of adding the filamentous fungus-derived protease and the microbe-derived lipase used in the present invention to milk is not particularly limited as long as the desired effect of the present invention is obtained. Examples of the addition timing include before or during incubation of milk; before, during, or after lactic acid fermentation of milk; and before, during, or after addition of the milk coagulating enzyme. From the viewpoint of improving the efficiency of contact between milk fat in the milk and the filamentous fungus-derived protease and the microbe-derived lipase to improve the production efficiency of a natural cheese, the filamentous fungus-derived protease and the microbe-derived lipase are preferably added before the addition of the milk coagulating enzyme.

The filamentous fungus-derived protease and the microbe-derived lipase may be added simultaneously or stepwise. In the case of adding the filamentous fungus-derived protease and the microbe-derived lipase stepwise, the order of addition of the filamentous fungus-derived protease and the microbe-derived lipase is not particularly limited, but as an example, the filamentous fungus protease can be added, and then the microbe-derived lipase can be added.

The addition of lactic acid bacteria for lactic acid fermentation can be performed at any timing, and the lactic acid bacteria can be preferably added at any stage before the addition of the milk coagulating enzyme.

The temperature at the time of adding the filamentous fungus-derived protease, the microbe-derived lipase, and the milk coagulating enzyme is not particularly limited as long as the desired effect of the present invention is obtained, and is, for example, 0 to 80°C, preferably 10 to 60°C, and further preferably 25 to 40°C. The time for which the filamentous fungus-derived protease and the microbe-derived lipase are allowed to act (aging period) can be set according to the type of a natural cheese, but is preferably 1 month or longer, more preferably 2 months or longer, and further preferably 2.5 months or longer, from the viewpoint of further increasing the released amount of short-chain fatty acids.

In the production method of a natural cheese of the present invention can appropriately include, in addition to the above steps, any step performed in the production of a natural cheese. Examples of the other steps include a sterilization step, a heating step, a cooling step, a pH adjustment step, a kneading step in hot water, a salt addition step, a molding step, an immersion step in a saturated saline solution, a drying step, and/or a hermetically packaging step. These steps can be easily selected by those skilled in the art according to the type of a natural cheese and the like.

2. Natural Cheese

A natural cheese of the present invention contains the microbe-derived lipase and the filamentous fungus-derived protease used in the production as residual components. The natural cheese of the present invention can be produced by the method described in "1. Production Method of Natural Cheese" above.

The natural cheese of the present invention contains a large amount of short-chain fatty acid (butyric acid) as compared with a natural cheese produced in the same manner except that the filamentous fungus-derived protease is not used. The specific content of the short-chain fatty acid (butyric acid) may vary depending on the type of cheese (water content) and the amounts of the microbe-derived lipase and the filamentous fungus-derived protease used, and is, for example, 0.8 $\mu$mol/g or more and preferably 4 $\mu$mol/g or more. From the viewpoint of further enhancing the flavor of a natural cheese, the specific content of the short-chain fatty acid (butyric acid) is preferably 6 $\mu$mol/g or more, more preferably 8 $\mu$mol/g or more, still more preferably 10 $\mu$mol/g or more, and even still more preferably 11 $\mu$mol/g or more.

The natural cheese of the present invention may be eaten as a natural cheese itself, or may be used as a material for a processed cheese. The processed cheese can be produced by appropriately heating and melting a natural cheese, blending an emulsifier (such as a citrate or a phosphate), an additive (such as oil, a preservative, a seasoning, a thickening stabilizer, a gelling agent, a pH adjuster, or a fragrance), and the like based on a known method. The natural cheese of the present invention can also be used as a material for a processed food product other than the processed cheese.

3. Short-Chain Fatty Acid Release Promoter in Natural Cheese Production

As described above, in the production method of the present invention using a microbe-derived lipase, the filamentous fungus-derived protease can enhance the effect of promoting short-chain fatty acid release. Therefore, the present invention also further provides a short-chain fatty acid release promoter in natural cheese production using a microbe-derived lipase, comprising a filamentous fungus-derived protease.

In the short-chain fatty acid release promoter of the present invention, the details or each enzyme, the method of use thereof, and the like are as described above in "1. Production Method of Natural Cheese".

The short-chain fatty acid release promoter of the present invention may contain other sitologically acceptable components in addition to each enzyme. Examples of the other components include excipients, disintegrants, antiseptics, preservatives, stabilizers, vitamins, minerals, sweeteners, and seasonings.

EXAMPLES

Hereinafter, the present invention will be specifically described by means of Examples; however, the present invention is not to be construed as being limited to the following Examples.

Used Enzyme

<Protease>

· Protease A "Amano" 2SD (hereinafter, referred to as "PR-A2SD"): Protease derived from filamentous fungus Aspergillus oryzae (A. oryzae) (manufactured by Amano Enzyme Inc.)
· Protin SD-NY10 (hereinafter, referred to as "SD-NY10"): Protease derived from bacterial Bacillus amyloliquefaciens (B. amyloliquefaciens) (manufactured by Amano Enzyme Inc.)

<Lipase>

· ItalaseC: Animal-derived lipase (DSM Food Specialties)
· G429M: Lipase corresponding to a polypeptide comprising an amino acid sequence in which amino acid sequences at the 1st to 15th positions are removed from an amino acid sequence shown in SEQ ID NO: 13

Activity Value Measurement Method

The method for measuring the activity values of the protease and the lipase is as follows.

<Protease Activity>

After 5 mL of a 0.6% (v/w) casein solution (0.05 mol/L of sodium hydrogen phosphate, pH 8.0 [in the case of PRSD-NY10] or pH 6.0 [in the case of PR-A2SD]) was warmed at 37°C for 10 minutes, 1 mL of a sample solution containing a protease was added, and the mixture was shaken. After this solution was left to stand at 37°C for 10 minutes, 5 mL of a trichloroacetic acid reagent (1.8% trichloroacetic acid, trichloroacetic acid containing 1.8% sodium acetate and 0.33 mol/L acetic acid [in the case of PRSD-NY10] or 0.44 mol/L [om the case of PR-A2SD]) was added, the mixture was shaken, and left to stand at 37°C for 30 minutes again, and the mixture was filtered. The first filtrate (3 mL) was removed, the next filtrate (2 mL) was weighed, 5 mL of a 0.55 mol/L sodium carbonate reagent and 1 mL of Folin's reagent (1 → 3) were added, and the mixture was shaken and left to stand at 37°C for 30 minutes. An absorbance AT of this solution (enzymatic reaction solution) at a wavelength of 660 nm was measured using water as a control.

Separately, an absorbance AB of a solution (blank) obtained by performing the same operation as in the above-described enzymatic reaction solution was measured, except that 1 mL of a sample solution containing a protease was weighed, 5 mL of a trichloroacetic acid reagent (1.8% trichloroacetic acid, trichloroacetic acid containing 1.8% sodium acetate and 0.33 mol/L acetic acid [in the case of PRSD-NY10] or 0.44 mol/L [in the case of PR-A2SD]) was added and shaken, 5 mL of a 0.6% (v/w) casein solution (0.05 mol/L sodium hydrogen phosphate, pH 8.0 [in the case of PRSD-NY10] or pH 6.0 [in the case of PR-A2SD]) was then added and shaken, and the mixture was left to stand at 37°C for 30 minutes.

An amount of an enzyme which causes an increase in colored materials by Folin's reagent corresponding to 1 μg of tyrosine per minute was defined as 1 unit (1 U).

Each of 1 mL, 2 mL, 3 mL, and 4 mL of a 1 mg/mL tyrosine standard stock solution (in 0.2 mol/L of hydrochloric acid) was weighed, and a 0.2 mol/L hydrochloric acid reagent was added thereto to make 100 mL. Each solution (2 mL) was weighed, 5 mL of a 0.55 mol/L sodium carbonate reagent and 1 mL of Folin's reagent (1 → 3) were added, and the mixture was shaken and left to stand at 37°C for 30 minutes. For these solutions, absorbances A1, A2, A3, and A4 at a wavelength of 660 nm were measured using, as a control, a solution obtained by weighing 2 mL of a 0.2 mol/L hydrochloric acid reagent and performing the same operation as described above. The absorbances A1, A2, A3, and A4 were plotted on the vertical axis and the amount (μg) of tyrosine in 2 mL of each solution was plotted on the horizontal axis to prepare a calibration curve, and the amount (μg) of tyrosine with respect to the absorbance difference of 1.

[Mathematical Formula 1]

$$\text{Protease activity (U/g, U/mL)} = (AT - AB) \times F \times 11/2 \times 1/10 \times 1/M$$

AT: Absorbance of enzymatic reaction solution
AB: Absorbance of blank
F: Amount (μg) of tyrosine with absorbance difference of 1 determined from tyrosine calibration curve
11/2: Conversion factor to total solution amount after stop of reaction
1/10: Conversion factor per minute of reaction time
M: Amount (g or mL) of sample in 1 mL of sample solution

<Lipase Activity>

In an emulsifying device, 70 mL of a 0.6 g/dL gum arabic solution (containing glycerin), 22.3 mL of tributyrin, and 329 mL of water were placed, and emulsified for 15 minutes (rotation: 3 times for 5 minutes, stop: 2 times for 5 minutes) under the condition of 14,500 ± 300 min$^{-1}$ to obtain a tributyrin substrate solution. Meanwhile, an appropriate amount of lipase was weighed, and diluted with cold water to prepare a sample solution. In a flat-bottom test tube, 30 mL of the tributyrin substrate solution was accurately weighed, and left to stand at 30 ± 0.5°C for 15 minutes. After standing, a pH electrode was immersed and stirred. (Thereafter, continuous stirring was performed during the operation.) This solution was adjusted to pH of 7.00 ± 0.05 using a 0.05 mol/L sodium hydroxide solution (for quantification).

Immediately after the pH adjustment, 2 mL of the sample solution was accurately added to start the reaction. After the start of the reaction, dropwise addition of a 0.05 mol/L sodium hydroxide solution (for quantification) was immediately started so as to maintain a pH of 7.00 ± 0.05, and while the dropwise addition was continued for exactly 5 minutes from the start of the reaction, a titration amount (V1 to V5 (mL)) was recorded every 1 minute.

An amount of an enzyme which causes an increase in butyric acid of 1.0 μmol per minute was defined as 1 unit (1 LU).

[Mathematical Formula 2]

$$\text{Lipase activity (LU/g)} = (V5 - V1)/4 \times 0.05 \times f \times 1000 \times D/2$$

V1: Titration amount (mL) after 1 minute from start of measurement
V5: Titration amount (mL) after 5 minutes from start of measurement
0.05: Normality of 0.05 mol/L sodium hydroxide solution
f: Factor of 0.05 mol/L sodium hydroxide solution
1000: Unit conversion factor (mmol → μmol)
D: Dilution factor (mL/g)
2: Added amount (mL) of sample solution

Confirmation of Short-Chain Fatty Acid Selectivity

After using 2 g of milk fat (in a state where fat globules are destructed with an emulsifier and milk fat is exposed) as a substrate and subjecting ItalaseC or G429M to a condition of acting in an amount of 3 LU per 1 g of milk fat at 40°C for 30 minutes, the released amounts of C4, C6, C8, C10, C12, C14, C16, C18, and C18:1 fatty acids were measured. As a result, it was confirmed that the released amount of the C4 fatty acid (butyric acid) was larger than the release amount of each of the C6, C8, C10, C12, C14, C16, C18, and C18: 1 fatty acids (compared on a molar basis).

Test Example 1

A material obtained by heat-sterilizing 225 kg (500 lb) of cow milk (milk fat: 2.9 wt%, milk protein: 3.3 wt%) at 73°C for 19 seconds was used. The sterilized cow milk was cooled to 34.4°C, 11.4 g of lactic acid bacteria (DSM CP-121) was added, and the mixture was stirred for about 1 hour until the pH decreased to 6.45. Next, 14.3 g of rennet (DSM Maxiren XDS BF; recombinant rennet in which bovine-derived chymosin gene is expressed in a yeast) was added thereto, stirring was stopped, and the mixture was left to stand still for 30 minutes. The formation of the card was confirmed, and the card was cut into a cube and heated to 41°C under stirring, and then the whey and the card were separated. An enzyme shown in Table 1 was added to 20 g of the card, and the mixture was thoroughly mixed, then heated to 65°C, molded, transferred to a container, and aged at 8°C. For the natural cheese (semi-hard type provolone cheese) obtained after aging for 3 months, the free fatty acid was analyzed according to the following procedure.

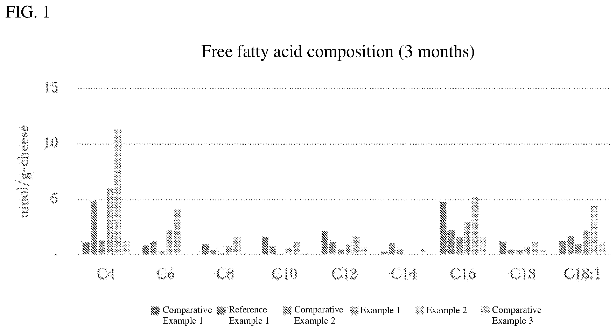

A slurry was prepared by thoroughly mixing 5 g of a sample (natural cheese) and 5 mL of a 0.2 M phosphate buffer solution (pH 6.8), and 1 g of the slurry, 0.05 mL of 2.5 M sulfuric acid, 0.05 mL of 0.1 M pelargonic acid (C9, internal standard), and 2.5 mL of chloroform were mixed by vortexing to extract a free fatty acid. The chloroform phase was recovered by centrifugation (7,000 rpm, 10 minutes), and the free fatty acid composition was analyzed by gas chromatography (Agilent, column: CP-Wax58). The results are shown in Table 1 and Fig. 1.

[Table 1]

| | | | Comparative Example 1 | Reference Example 1 | Comparative Example 2 | Example 1 | Example 2 | Comparative Example 3 |
|---|---|---|---|---|---|---|---|---|
| Protease | | | - | - | - | A. oryzae origin | A. oryzae origin | B. amyloliquefaciens origin |
| | | | | | | PR-A2SD | PR-A2SD | SD-NY10 |
| | Added amount | w/w-card | - | - | - | 500 ppm | 1000 ppm | 1000 ppm |
| | | U/g-card | | | | 50 | 100 | 70 |
| Lipase | | | - | Animal origin | Microbe-derived -variant | Microbe-derived -variant | Microbe-derived -variant | Microbe-derived -variant |
| | | | | ItalaseC | G429M | G429M | G429M | G429M |
| | Added amount | w/w-card | - | 700 ppm | 700 ppm | 700 ppm | 700 ppm | 700 ppm |
| | | LU/g-card | | 0.01 | 0.509 | 0.509 | 0.509 | 0.509 |
| Fatty acid released amount µmol/g-cheese | C4 | | 1.13 | 4.9 | 1.29 | 6.06 | 11.35 | 1.19 |
| | C6 | | 0.91 | 1.17 | 0.28 | 2.33 | 4.16 | 0.23 |
| | C8 | | 0.95 | 0.42 | 0.13 | 0.74 | 1.59 | 0.16 |
| | C10 | | 1.6 | 0.77 | 0.16 | 0.6 | 1.15 | 0.22 |
| | C12 | | 2.21 | 1.13 | 0.48 | 0.95 | 1.68 | 0.67 |
| | C14 | | 0.29 | 1.05 | 0.48 | 0.02 | 0.08 | 0.52 |
| | C16 | | 4.8 | 2.29 | 1.6 | 2.99 | 5.17 | 1.59 |
| | C18 | | 1.18 | 0.49 | 0.41 | 0.69 | 1.11 | 0.4 |
| | C18:1 | | 1.19 | 1.67 | 0.98 | 2.33 | 4.43 | 1.07 |

As shown in Table 1, when an animal-derived lipase was used in the production of a natural cheese (Reference Example 1), a short-chain fatty acid (butyric acid having 4 carbon atoms) was sufficiently released, but when the microbe-derived lipase was used (Comparative Example 2), a short-chain fatty acid could be confirmed only to such an extent when a lipase was not used (Comparative Example 1). On the other hand, when the filamentous fungus-derived protease was used in combination with the microbe-derived lipase (Examples 1 and 2), the amount of the released short-chain fatty acid increased as compared with Comparative Example 2, and the effect thereof was enhanced depending on the added amount of the filamentous fungus-derived protease. When a bacteria-derived protease was used instead of the filamentous fungus-derived protease, the effect of increasing the amount of the short-chain fatty acid was not observed (Comparative Example 3).

Test Example 2

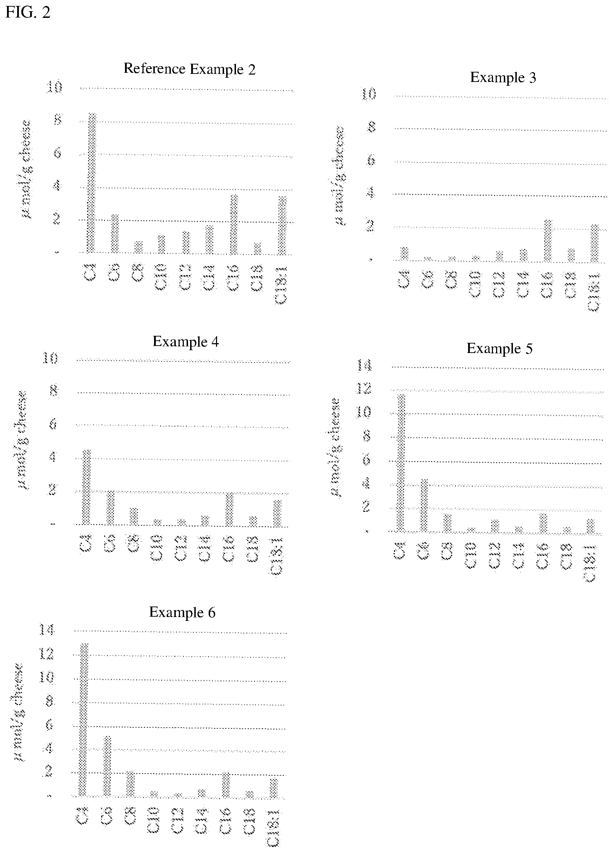

A 3.8-L commercially available cow milk (milk fat: 3.5 wt%, milk protein: 3 wt%) was warmed to 34°C, and 1 g of mesophilic lactic acid bacteria (Mesophilic Direct Set Cheese Culture (C101)), 1 g of thermophilic lactic acid bacteria (Thermophilic Direct Set Cheese Culture (C201)), and an enzyme shown in Table 2 were added thereto. After stirring for 30 minutes, 0.24 g of rennet (DSM Maxiren XDS BF) was added and the mixture was left to stand still for 35 minutes. The formation of the card was confirmed, and the card was roughly cut and heated to 41°C over 30 minutes under stirring. After the pH decreased to 5.9, the whey and the card were separated. The collected card was immersed at 38°C for 1 hour, the card was then cut into a cube, and after confirming that the pH decreased to 5.2, the card was thoroughly mixed with 11.8 g of dietary salt. Subsequently, the card was treated in hot water at 70°C until the stretch was sufficiently obtained, then taken out of the hot water, and molded and immersed in cold water for 30 minutes. The card was further immersed in a saturated saline solution at 10°C overnight, and the cheese taken out was dried at room temperature for 2 hours, hermetically packaged, and aged at 8°C. For the natural cheese (semi-hard type provolone cheese) obtained after aging for 3 months, the free fatty acid was analyzed in the same manner as in Test Example 1. The results are shown in Table 2 and Fig. 2.

The flavor of the obtained natural cheese was sensory evaluated based on the following criteria. The short-chain fatty acid represents butyric acid having 4 carbon atoms. The results are shown in Table 2.

+++: The flavor derived from a short-chain fatty acid is strongly felt.
++: The flavor derived from a short-chain fatty acid is felt.
+: The flavor derived from a short-chain fatty acid is weakly felt.
-: The flavor derived from a short-chain fatty acid is not felt.

The shape retention of the obtained natural cheese was evaluated based on the following criteria using, as an index of the shape retention, the degree of change in shape after aging based on the shape of the natural cheese before aging. The results are shown in Table 2.

⊙: The shape did not change (the shape was retained).
O: The change in shape was such an extent that the shape was slightly collapsed (the shape was almost retained).
Δ: The change in shape was recognized to such an extent that the shape was slightly collapsed (the shape was barely retained).
×: The change in shape was severe (the shape was not retained).

[Table 2]

| | | | Reference Example 2 | Example 3 | Example 4 | Example 5 | Example 6 |
|---|---|---|---|---|---|---|---|
| Protease | | | - | A. oryzae origin | A. oryzae origin | A. oryzae origin | A. oryzae origin |
| | | | | PR-A2SD | PR-A2SD | PR-A2SD | PR-A2SD |
| | Added amount | w/w-cow milk | - | 1.3 ppm | 2.6 ppm | 0.26 ppm | 1.3 ppm |
| | | U/kg-cow milk | | 130 | 260 | 26 | 130 |
| | | U/g-milk fat | | 3.7 | 7.4 | 0.7 | 3.7 |
| | | U/g-milk protein | | 4.3 | 8.7 | 0.9 | 4.3 |
| Lipase | | | Animal origin | Microbe-derived -variant | Microbe-derived -variant | Microbe-derived -variant | Microbe-derived -variant |
| | | | ItalaseC | G429M | G429M | G429M | G429M |
| | Added amount | w/w-cow milk | 66 ppm | 66 ppm | 66 ppm | 200 ppm | 200 ppm |
| | | LU/kg- cow milk | 1 | 48 | 48 | 146 | 146 |
| | | LU/g-milk fat | 0.03 | 1.4 | 1.4 | 4.2 | 4.2 |
| | | LU/g-milk protein | 0.03 | 1.6 | 1.6 | 4.9 | 4.9 |
| Fatty acid released amount μmol/g-cheese | C4 | | 8.53 | 0.83 | 4.55 | 11.63 | 13.03 |
| | C6 | | 2.37 | 0.19 | 2.1 | 4.49 | 5.2 |
| | C8 | | 0.76 | 0.23 | 1.05 | 1.55 | 2.19 |
| | C10 | | 1.18 | 0.34 | 0.34 | 0.4 | 0.46 |
| | C12 | | 1.43 | 0.67 | 0.39 | 1.12 | 0.35 |
| | C14 | | 1.77 | 0.78 | 0.6 | 0.55 | 0.68 |
| | C16 | | 3.62 | 2.63 | 2.03 | 1.71 | 2.21 |
| | C18 | | 0.74 | 0.88 | 0.63 | 0.53 | 0.62 |
| | C18:1 | | 3.57 | 2.33 | 1.63 | 1.3 | 1.74 |
| Flavor | | | +++ | + | + | +++ | +++ |
| Shape retention | | | ⊙ | Δ | Δ | ⊙ | Δ |

As is apparent from Table 2, when the filamentous fungus-derived protease was used in combination with the microbe-derived lipase (Examples 3 to 6), the flavor derived from a short-chain fatty acid could be felt, and particularly in Examples 5 and 6, the flavor could be remarkably felt. Although not shown in the data, in the natural cheese produced by performing the same step as in Examples 3 to 6 except that the filamentous fungus-derived protease was not used, the flavor derived from a short-chain fatty acid was not felt. The higher the added amount of the microbe-derived lipase (Examples 5 and 6 with respect to Examples 3 and 4) and the higher the added amount of the filamentous fungus-derived protease (Example 4 with respect to Example 3, Example 6 with respect to Example 5), the higher the released amount of the short-chain fatty acid, but in Example 5 in which the added amount of the filamentous fungus-derived protease was suppressed, remarkably excellent shape retention was observed.
SEQ ID NO: 1 is a P261A variant of lipase LIP1 comprising a signal peptide derived from Candida cylindracea.
SEQ ID NO: 2 is a P261F variant of lipase LIP1 comprising a signal peptide derived from Candida cylindracea.
SEQ ID NO: 3 is a P261L variant of lipase LIP1 comprising a signal peptide derived from Candida cylindracea.
SEQ ID NO: 4 is a P261S variant of lipase LIP1 comprising a signal peptide derived from Candida cylindracea.
SEQ ID NO: 5 is an L322W variant of lipase LIP1 comprising a signal peptide derived from Candida cylindracea.
SEQ ID NO: 6 is a F360L variant of lipase LIP1 comprising a signal peptide derived from Candida cylindracea.
SEQ ID NO: 7 is an S380I variant of lipase LIP1 comprising a signal peptide derived from Candida cylindracea.

SEQ ID NO: 8 is an S380L variant of lipase LIP1 comprising a signal peptide derived from Candida cylindracea.

SEQ ID NO: 9 is an L425W variant of lipase LIP1 comprising a signal peptide derived from Candida cylindracea.

SEQ ID NO: 10 is an L428F variant of lipase LIP1 comprising a signal peptide derived from Candida cylindracea.

SEQ ID NO: 11 is an L428N variant of lipase LIP1 comprising a signal peptide derived from Candida cylindracea.

SEQ ID NO: 12 is a G429F variant of lipase LIP1 comprising a signal peptide derived from Candida cylindracea.

SEQ ID NO: 13 is a G429M variant of lipase LIP1 comprising a signal peptide derived from Candida cylindracea.

SEQ ID NO: 14 is a G429I variant of lipase LIP1 comprising a signal peptide derived from Candida cylindracea.

SEQ ID NO: 15 is a V549F variant of lipase LIP1 comprising a signal peptide derived from Candida cylindracea.

**Claims**

1.    A production method of a natural cheese, comprising a step of causing a microbe-derived lipase and a filamentous fungus-derived protease to act on milk.

2.    The production method according to claim 1, wherein the microbe-derived lipase comprises at least any one of polypeptides shown in (1) to (3) below:

    (1) a polypeptide comprising an amino acid sequence in which amino acid sequences at the 1st to 15th positions are removed from an amino acid sequence shown in any one of SEQ ID NOs: 1 to 15 or each of amino acid sequence shown in SEQ ID NOs: 1 to 15;
    (2) a polypeptide comprising an amino acid sequence in which amino acid sequences at the 1st to 15th positions are removed from an amino acid sequence shown in any one of SEQ ID NOs: 1 to 15 or each of amino acid sequence shown in SEQ ID NOs: 1 to 15 obtained by substituting, adding, inserting, or deleting one or several amino acid residues and having short-chain fatty acid selectivity; and
    (3) a polypeptide having 70% or more sequence identity to an amino acid sequence in which amino acid sequences at the 1st to 15th positions are removed from an amino acid sequence shown in any one of SEQ ID NOs: 1 to 15 or each of amino acid sequence shown in SEQ ID NOs: 1 to 15, and having short-chain fatty acid selectivity.

3.    The production method according to claim 1 or 2, wherein a used amount of the microbe-derived lipase is 1 LU or more per 1 g of milk fat in the milk.

4.    The production method according to any one of claims 1 to 3, wherein a used amount of the filamentous fungus-derived protease per 1 LU of the microbe-derived lipase is 0.02 U or more.

5.    The production method according to any one of claims 1 to 4, wherein a used amount of the filamentous fungus-derived protease is 0.3 U or more per 1 g of milk protein in the milk.

6.    The production method according to claim 5, wherein the used amount of the filamentous fungus-derived protease is 20 U or less per 1 g of milk protein in the milk.

7.    The production method according to any one of claims 1 to 6, wherein the filamentous fungus-derived protease is a protease derived from the genus Aspergillus.

8.    A natural cheese comprising a microbe-derived lipase and a filamentous fungus-derived protease.

9.    A short-chain fatty acid release promoter in natural cheese production using a microbe-derived lipase, comprising a filamentous fungus-derived protease.

10.    A production method of a processed cheese product, comprising:

    a step of producing a natural cheese by the production method according to any one of claims 1 to 7; and
    a step of processing the natural cheese.

11.    A processed cheese product produced by the production method according to claim 10.

FIG. 1

## Free fatty acid composition (3 months)

FIG. 2

Reference Example 2

Example 3

Example 4

Example 5

Example 6

## INTERNATIONAL SEARCH REPORT

| International application No. |
| --- |
| **PCT/JP2022/004515** |

### A. CLASSIFICATION OF SUBJECT MATTER

*A23C 19/032*(2006.01)i; *A23C 19/068*(2006.01)i; *C12N 9/20*(2006.01)i; *C12N 9/62*(2006.01)i
FI: A23C19/032; A23C19/068; C12N9/20 ZNA; C12N9/62

According to International Patent Classification (IPC) or to both national classification and IPC

### B. FIELDS SEARCHED

Minimum documentation searched (classification system followed by classification symbols)

A23C19/032; A23C19/068; C12N9/20; C12N9/62

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); GenBank/EMBL/DDBJ/GeneSeq; UniProt/GeneSeq; SwissProt/GeneSeq

### C. DOCUMENTS CONSIDERED TO BE RELEVANT

| Category* | Citation of document, with indication, where appropriate, of the relevant passages | Relevant to claim No. |
| --- | --- | --- |
| Y | JP 2002-165558 A (KRAFT FOODS HOLDINGS INC) 11 June 2002 (2002-06-11) paragraphs [0064]-[0069], fig. 1 | 1-11 |
| Y | JP 2007-519410 A (DSM IP ASSETS B.V.) 19 July 2007 (2007-07-19) paragraphs [0011], [0018] | 1-11 |
| Y | JP 8-509366 A (NOVO NORDISK AS) 08 October 1996 (1996-10-08) p. 13, lines 4-6, example 4 | 1-11 |
| Y | WO 2015/087833 A1 (AMANO ENZYME INC) 18 June 2015 (2015-06-18) claims, paragraph [0017], examples | 1-11 |
| Y | WO 2017/211930 A1 (DSM IP ASSETS B.V.) 14 December 2017 (2017-12-14) claims, examples | 1-11 |

☐ Further documents are listed in the continuation of Box C.　　☑ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **24 March 2022** | **05 April 2022** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**

International application No.

**PCT/JP2022/004515**

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

INTERNATIONAL SEARCH REPORT
Information on patent family members

International application No.

**PCT/JP2022/004515**

| Patent document cited in search report | | | Publication date (day/month/year) | Patent family member(s) | | | Publication date (day/month/year) |
|---|---|---|---|---|---|---|---|
| JP | 2002-165558 | A | 11 June 2002 | US fig. 1 EP | 6406724 1186244 | B1 A2 | |
| JP | 2007-519410 | A | 19 July 2007 | WO p. 6, lines 1-4, example 4 US EP | 2005/074695 2007/0160711 1705998 | A1 A1 A1 | |
| JP | 8-509366 | A | 08 October 1996 | WO p. 1, lines 22-25, example 4 | 1994/025580 | A1 | |
| WO | 2015/087833 | A1 | 18 June 2015 | US claims, paragraph [0049], examples EP | 2016/0319259 3081644 | A1 A1 | |
| WO | 2017/211930 | A1 | 14 December 2017 | (Family: none) | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 61135541 A
- US 20040001819
- JP 2011512809 W
- JP 2003524386 W
- JP 2004517639 W
- WO 2015087833 A
- WO 2017211930 A

**Non-patent literature cited in the description**

- **TATIANA A. TATSUSOVA ; THOMAS L. MADDEN.** *FEMS Microbiol. Lett.,* 1999, vol. 174, 247-250